Europäisches Patentamt

(19) European Patent Office

Office européen des brevets

(11) Publication number: **0 032 758**
**B1**

(12) EUROPEAN PATENT SPECIFICATION

(45) Date of publication of patent specification: **14.03.84**

(21) Application number: **81200008.1**

(22) Date of filing: **06.01.81**

(51) Int. Cl.³: **C 07 C 179/035,**
**C 07 C 178/00**

(54) **A method for the prevention of disturbances and/or the effects of disturbances in the preparation of hydrocarbon hydroperoxides by oxidation of hydrocarbons with molecular oxygen.**

(30) Priority: **21.01.80 NL 8000363**

(43) Date of publication of application:
**29.07.81 Bulletin 81/30**

(45) Publication of the grant of the patent:
**14.03.84 Bulletin 84/11**

(84) Designated Contracting States:
**DE FR GB NL**

(56) References cited:
**FR - A - 2 287 429**
**US - A - 2 867 666**

(73) Proprietor: SHELL INTERNATIONALE RESEARCH
MAATSCHAPPIJ B.V.
Carel van Bylandtlaan 30
NL-2596 HR Den Haag (NL)

(72) Inventor: Van der Weijst, Ludovicus B. J. O.
Buitendijk - Oost 25
NL-4791 RK Klundert (NL)
Inventor: De Vries, Enno Boelo
Badhuisweg 3
NL-1031 CM Amsterdam (NL)
Inventor: Heiszwolf, Gerard Johan
Badhuisweg 3
NL-1031 CM Amsterdam (NL)

(74) Representative: Puister, Antonius Tonnis, Mr. et al,
P.O. Box 302
NL-2501 CH The Hague (NL)

Courier Press, Leamington Spa, England.

**0 032 758**

A method for the prevention of disturbances and/or the effects of disturbances in the preparation of hydrocarbon hydroperoxides by oxidation of hydrocarbons with molecular oxygen

The invention relates to a method for the prevention of disturbances and/or the effects of disturbances in the preparation of hydrocarbon hydroperoxides by oxidation of hydrocarbons with molecular oxygen.

It is known that hydroperoxides of hydrocarbons, such as for example isobutane, cyclohexane, cumene and ethyl benzene, can be prepared by passing oxygen or an oxygen-containing gas through the relevant hydrocarbon at elevated temperature. To shorten the induction period and improve the selectivity the reaction is usually carried out in the presence of a basic substance.

U.S. patent specification 2,632,772 mentions as suitable basic substances, inter alia, the hydroxides and carbonates of alkali metals, the oxides and hydroxides of alkaline earth metals, the normal phosphates of said metals, and ammonia. In U.K. patent specification 713,138 secondary or tertiary amines, in particular pyridine, are used and in Netherlands patent specification 6810123 the use of alkali metal pyrophosphates is recommended, preferably in such quantities that the alkali metal content of the oxidation mixture is 0.1—100 ppmw.

It is known, for example, from Netherlands patent application 7511955 that in the oxidation of hydrocarbons with a molecular oxygen-containing gas a greatly accelerated decomposition of the hydroperoxide (known as a runaway) may suddenly occur. A possible cause thereof may be that small quantities of substances which catalyze the decomposition of the hydroperoxide find their way into the reaction system. Since the decomposition of the hydroperoxide is attended by a high degree of heat development, it is of great importance that the rise in temperature caused by the accelerated decomposition is controlled as soon as possible, since otherwise an explosion might take place.

In Netherlands patent application 7511955 this problem is discussed with reference to an example regarding the oxidation of cumene. According to said patent application, in the processes in which cumene is oxidized not a single effective method was known to control a dangerous rise in temperature in time and use was made only of membrane cooling surfaces having limited effectiveness. Netherlands patent application 7511955 therefore proposes a process for the prevention of disturbances and/or the effects of disturbances in the oxidation of hydrocarbons in the liquid phase under pressure with oxygen-containing gases, in which process water is introduced into the reaction vessel in an intensive way, preferably in a quantity which is necessary to cool the liquid hydrocarbon to below the boiling point at atmospheric pressure of the hydroazeotrope of the hydrocarbon to be oxidized or to a temperature at which no uncontrolled decomposition of the hydroperoxide takes place. The water is preferably introduced in a period of 0.5—5 minutes. It is, of course, obvious that said process, in which large quantities of water are sprayed into the reactor, involves great practical drawbacks.

It has now been found that a sudden undesirable rise in temperature in the oxidation of hydrocarbons with molecular oxygen can be controlled in a considerably less drastic manner. The invention relates to a method for the prevention of disturbances and/or the effects of disturbances in the preparation of hydrocarbon hydroperoxides by oxidation of hydrocarbons with molecular oxygen or a molecular oxygen-containing gas at elevated temperature, characterized in that if an uncontrolled rise in temperature occurs in performing the oxidation, $NH_3$ is introduced into the reaction mixture. If the reaction is already being carried out in the presence of a basic substance, an extra quantity of the basic substance $NH_3$ should be added. It is surprising that by adding a usually small (extra) quantity of $NH_3$ to the reaction mixture at the moment when the uncontrolled rise in temperature starts, the latter can be checked.

The ammonia can be introduced into the reaction mixture by addition to the hydrocarbon feed, to the oxygen or the oxygen-containing gas or to the reaction mixture itself. In any case care should be taken that the ammonia is completely mixed with the reaction mixture as quickly as possible, preferably within 5 minutes. This can be effected by stirring vigorously, using stirring systems present in the reactor. In order to ensure that the mixing is completed rapidly, the ammonia is preferably added to the oxygen or the oxygen-containing gas. In this case the ammonia is vaporous at room temperature or at least at the temperature at which the oxygen or the oxygen-containing gas is passed into the reaction mixture. Very good results can be obtained by adding for example 1—300, preferably 2—80 ppmw of $NH_3$, based on the weight of the reaction mixture present in the reactor, to the oxygen or the oxygen-containing gas. The use of smaller or larger quantities, however, is not excluded.

Immediately after the ammonia has been introduced into the reaction mixture, the rise in temperature discontinues. To accelerate a reduction in temperature in the event of an uncontrolled rise in temperature, it will be preferred also to start up cooling systems present in the reactor. Consequently, the reaction mixture can optionally be cooled to a temperature at which practically no further reactions take place. In addition, the supply of oxygen or of the oxygen-containing gas will preferably also be reduced or closed. The reaction mixture which is present in the reactor and is optionally cooled can then be passed to the next step of the process and be processed further in the usual manner. Subsequently, the reactor can be filled with fresh hydrocarbon and the oxidation can be continued. However, in a continuous process, after the addition of the ammonia it is in principle possible to continue the passing through of the oxygen or the oxygen-containing gas. Of course, measures must then be taken to remove the cause of the runaway, for example by switching over to another feed. In such a case, especially if the reaction mixture is not cooled, it may be necessary to repeat the addition of ammonia once or several times when taking the relevant measures, if an uncontrolled rise in temperature again takes place.

The method according to the invention is particularly suitable for use in the preparation of hydroperoxides of tertiary alkanes, cycloalkanes and aralkanes. Said hydrocarbons preferably contain 4—20 carbon atoms. The aralkanes may contain one or more aromatic rings optionally substituted with one or more alkyl groups. Examples of suitable hydrocarbons are isobutane, isopentane, isohexane, 2,3-dimethylbutane, cumene, ethylbenzene, ethyltoluene, ethylnaphthalene, cyclopentane, cyclohexane, methylcyclohexane and cyclododecane. The temperature at which the oxidation is carried out depends on the hydrocarbon to be oxidized and is mostly between 80 and 160°C. The oxidation of, for example, cumene is generally carried out at a temperature between 80 and 140°C, and in the oxidation of ethylbenzene a temperature between 135 and 160°C is mostly used. The oxidation is mostly carried out at a pressure between 1 and 70 bar abs.

The moment when an uncontrolled rise in temperature takes place can easily be determined by any operator. The oxidation of, for example, ethylbenzene can very suitably be carried out at a constant temperature of 150°C. If the temperature suddenly rises to, for example, 152°C without apparent cause, this may signify that undesirable decomposition of the hydroperoxide is taking place and that the method according to the invention must be used.

Consequently, the invention also relates to a process for the preparation of a hydrocarbon hydroperoxide in which oxygen or a molecular oxygen-containing gas is passed through a hydrocarbon at elevated temperature, characterized in that in order to prevent disturbances and/or the effects of disturbances ammonia is introduced into the reaction mixture if in the course of the reaction an uncontrolled rise in temperature takes place.

Example I

A reactor in which a distributor for the introduction of a gas and a stirrer had been installed, was charged with 1 litre of ethylbenzene to which 1.5 ppm of sulphur in the form of 2,5-dimethylthiophene had been added. Under the conditions in which the oxidation is carried out, the latter compound can catalyze the decomposition of the hydroperoxide formed. At a temperature of 150°C and a pressure of 3 bar abs., 80 l/h of a mixture consisting of air and nitrogen was passed through the ethylbenzene. The ratio between the quantities of air and nitrogen was adjusted in such a way that the oxygen concentration in the off-gas was 4% by volume. After 90 minutes the temperature suddenly started to rise and was 152°C after 15 minutes (total reaction time 105 minutes). At this moment 10 ppm of $NH_3$ (based on the weight of the reaction mixture) were added to the mixture of air and nitrogen. In spite of the fact that no measures were taken to reduce the temperature of the reaction mixture and that the passing through of the oxygen-containing gas was continued, the temperature immediately started to fall and reached the original value of 150°C after 10 minutes (reaction time 115 minutes). After a reaction time of 125 and 150 minutes quantities of 10 ppm of $NH_3$ were once more added. The results are summarized in the following Table A which also states the phenol content of the reaction mixture, a measure for the decomposition of the hydroperoxide.

TABLE A

| Addition of 10 ppm $NH_3$ | Reaction time in min. | Temperature, °C | Phenol content, % by wt | Ethylbenzene hydroperoxide, % by wt |
|---|---|---|---|---|
| | 90 | 150 | 0.06 | 7.3 |
| → | 105 | 152 | 0.22 | 8.3 |
| | 115 | 150 | 0.32 | 9.0 |
| → | 125 | 152 | 0.72 | 8.9 |
| | 135 | 151 | — | — |
| → | 150 | 153 | 1.6 | 8.3 |
| | 180 | 152 | 2.1 | 7.5 |

# 0 032 758

Table A shows that by the addition of very small quantities of a base it is possible to check the undesirable rise in temperature for a long period of time and to limit the decomposition of the hydroperoxide. In that period the cause of the rise in temperature can be removed, for example by switching over to another feed.

Table B summarizes the results of an experiment in which the oxidation of ethylbenzene was repeated in the manner described in this Example, but no $NH_3$ was added. No measures were taken to reduce the temperature of the reaction mixture and the passing through of the oxygen-containing gas was continued during the entire test.

## TABLE B

| Reaction time in min. | Temperature °C | Phenol content, % by wt | Ethylbenzene hydroperoxide content, % by wt |
|---|---|---|---|
| 60 | 150 | $\leqslant 0.05$ | 5.3 |
| 90 | 150 | $\leqslant 0.05$ | 8.7 |
| 105 | 151 | 0.22 | 10.0 |
| 115 | 153 | 0.85 | 9.4 |
| 125 | 155 | 1.8 | 7.3 |
| 135 | 153 | 2.7 | 5.5 |
| 150 | 150 | 3.4 | 3.8 |
| 180 | 149 | 4.1 | 1.6 |

Table B shows that if no $NH_3$ is added, the greater part of the ethylbenzene hydroperoxide decomposes.

## Example II

In the manner described in Example I ethylbenzene, to which 1.5 ppm of sulphur had been added as 2,5-dimethylthiophene, was oxidized at a temperature of 150°C and a pressure of 3 bar abs. After a reaction time of 90 minutes the temperature started to rise and was 152°C after a reaction time of 106 minutes. At this moment 10 ppm of $NH_3$ (based on the weight of the reaction mixture) were added to the mixture of air and nitrogen and the reaction mixture was cooled. The results are summarized in Table C.

## TABLE C

| Addition of 10 ppm $NH_3$ | Reaction time in min. | Temperature, °C | Phenol content, % by wt | Ethylbenzene hydroperoxide content, % by wt |
|---|---|---|---|---|
| | 90 | 150 | 0.06 | 7.6 |
| $\rightarrow$ | 106 | 152 | 0.39 | 8.6 |
| | 121 | 141 | 0.67 | 9.0 |
| ⌐ | 151 | 142 | 1.2 | 8.7 |
| | 181 | 138 | 1.9 | 7.8 |

Table C shows that by a single addition of a very small quantity of a base and cooling of the reaction mixture the undesirable rise in temperature can be controlled and the decomposition of the hydroperoxide can be limited.

4

Example III

In the manner described in Example I ethylbenzene, to which 1.5 ppm of sulphur had been added as 2,5-dimethylthiophene, was oxidized at a temperature of 150°C and a pressure of 3 bar abs. After a reaction time of 84 minutes the temperature began to rise and was 152°C after 95 minutes. At this moment 20 ppm of $NH_3$ were added to the gas stream and the reactor was cooled. Three minutes after the $NH_3$-injection the passing through of the mixture of air and nitrogen was discontinued and a weak nitrogen stream was passed through the reactor instead. The results are summarized in Table D.

TABLE D

| Addition of 20 ppm $NH_3$ | Reaction time in min. | Temperature, °C | Phenol content, % by wt | Ethylbenzene hydroperoxide content, % by wt |
|---|---|---|---|---|
| | 90 | 151 | 0.34 | 5.9 |
| → | 95 | 152 | 0.54 | 6.1 |
| | 98 | 150 | — | — |
| | 110 | 140 | 0.52 | 6.0 |
| | 140 | 140 | 0.54 | 5.7 |
| | 170 | 140 | 0.55 | 5.4 |

Table D shows that the formation of phenol by decomposition of the hydroperoxide can be practically stopped by adding a small quantity of a base, passing no further oxygen-containing gas through the reaction mixture and cooling the reaction mixture.

Repetition of the test with 10 ppm of $NH_3$ yielded practically the same results.

Table E summarizes the results of an experiment in which the oxidation of ethylbenzene was repeated in the manner described in this Example, but no $NH_3$ was added. After a reaction time of 95 minutes the reaction mixture was cooled and after 97 minutes instead of the mixture of air and nitrogen a weak nitrogen stream was passed through the reaction mixture.

TABLE E

| Reaction time in min. | Temperature, °C | Phenol content, % by wt | Ethylbenzene hydroperoxide content, % by wt |
|---|---|---|---|
| 60 | 150 | ⩽0.05 | 4.6 |
| 90 | 151 | 0.42 | 6.1 |
| 95 (cooling) | 152 | 0.72 | 6.0 |
| 97 (no air) | | — | — |
| 100 | 154 | — | — |
| 106 | 153 | — | — |
| 115 | 145 | — | — |
| 120 | 140 | 1.8 | 2.5 |
| 180 | 140 | 2.0 | 1.3 |
| 240 | 140 | 2.1 | 0.9 |

**0 032 758**

Table E shows that in spite of cooling and closure of the air stream the decomposition of the hydroperoxide continues.

Table F summarizes the results of an experiment which was carried out in the same manner and in which no $NH_3$ was added, the reaction mixture was not cooled and after a reaction time of 110 minutes instead of the mixture of air and nitrogen a weak nitrogen stream was passed through the reaction mixture.

TABLE F

| Reaction time in min. | Temperature, °C | Phenol content, % by wt | Ethylbenzene hydroperoxide content, % by wt |
|---|---|---|---|
| 90 | 150 | ⩽0.05 | 8.3 |
| 110 (no air) | 152 | 0.41 | 9.4 |
| 120 | 157 | 1.4 | 5.8 |
| 135 | 151 | 1.9 | 3.4 |
| 150 | 149 | 2.2 | 2.5 |
| 180 | 150 | 2.3 | 1.6 |

Table F shows that in spite of the closure of the air stream the decomposition of the hydroperoxide continues.

Example IV

In the manner described in Example I ethylbenzene, to which 1.5 ppm of sulphur had been added as 2,5-dimethylthiophene, was oxidized at a temperature of 150°C. After a reaction time of 80 minutes the temperature started to rise and since the reaction mixture was slightly heated in order to compensate for the loss of heat to the surroundings, the temperature reached a value of 162°C after a reaction time of 120 minutes. At this moment 10 ppm of $NH_3$ were injected into the mixture of air and nitrogen and the temperature immediately started to fall. The reaction mixture was cooled to 140°C in 20 minutes and maintained at 140°C for a further hour. During the cooling and the subsequent hour a constant stream of a mixture of air and nitrogen was passed through the reaction mixture. The ratio between the quantities of air and nitrogen was adjusted in such a way that the oxygen concentration in the off-gas was 4% by volume. The results are summarized in Table G.

TABLE G

| Addition of 10 ppm $NH_3$ | Reaction time in min. | Temperature, °C | Phenol content, % by wt | Ethylbenzene hydroperoxide content, % by wt |
|---|---|---|---|---|
| | 60 | 150 | ⩽0.05 | 4.7 |
| | 80 | 151 | — | — |
| | 90 | 152 | 0.14 | 7.5 |
| | 100 | 157 | 0.40 | 7.8 |
| | 110 | 161 | 1.2 | 6.8 |
| → | 120 | 162 | 2.3 | 4.3 |
| | 140 | 139 | 2.5 | 4.2 |
| | 170 | 140 | 2.5 | 4.2 |
| | 200 | 140 | 2.7 | 4.2 |

6

This test shows that it is also possible to regain control of a runaway up to a relatively high temperature (162°C) by the addition of a small quantity of base.

Example V

In the manner described in Example I ethylbenzene, to which 1.5 ppm of sulphur had been added as 2,5-dimethylthiophene, was oxidized at a temperature of 150°C. To the mixture of air and nitrogen 0.25 nml of gaseous NH₃ was added every 2.5 minutes, so that after 2 hours 10 ppm, based on the ethylbenzene, had been added. Table H shows that a runaway cannot be prevented by this semi-continuous addition of NH₃.

TABLE H

| Reaction time in min. | Temperature, °C | Phenol content, % by wt | Ethylbenzene hydroperoxide content, % by wt |
|---|---|---|---|
| 60 | 150 | ⩽0.05 | 4.3 |
| 90 | 150 | ⩽0.05 | 7.0 |
| 120 | 154 | 0.44 | 7.6 |
| 150 | 152 | 2.6 | 2.1 |

**Claims**

1. A method for the prevention of disturbances and/or the effects of disturbances in the preparation of hydrocarbon hydroperoxides by oxidation of hydrocarbons with molecular oxygen or a molecular oxygen-containing gas at elevated temperature characterized in that if an uncontrolled rise in temperature occurs in performing the oxidation, NH₃ is introduced into the reaction mixture.

2. A method as claimed in claim 1, characterized in that the NH₃ is added to the oxygen or the oxygen-containing gas.

3. A method as claimed in claim 2, characterized in that the NH₃ is vaporous at room temperature or at least at the temperature at which the oxygen or the oxygen-containing gas is introduced into the reaction mixture.

4. A method as claimed in claim 1, characterized in that the quantity of NH₃ is between 1 and 300, in particular between 2 and 80 ppmw, based on the weight of the reaction mixture present in the reactor.

5. A method as claimed in claims 1—4, characterized in that in the event of the uncontrolled rise in temperature cooling systems present in the reactor are also started up and/or the supply of oxygen or the oxygen-containing gas is reduced or closed.

**Revendications**

1. Un procédé pour la prévention de perturbations et/ou des effets de perturbations dans la préparation d'hydroperoxydes d'hydrocarbures par oxydation d'hydrocarbures par l'oxygène moléculaire ou un gaz contenant de l'oxygène moléculaire à température élevée, caractérisé en ce que si une élévation incontrôlée de température se produit durant la conduite de l'oxydation, on introduit de l'ammoniac dans le mélange réactionnel.

2. Un procédé selon la revendication 1, caractérisé en ce que l'ammoniac est ajouté à l'oxygène ou au gaz contenant de l'oxygène.

3. Un procédé selon la revendication 2, caractérisé en ce que l'ammoniac est à l'état de vapeur à la température ambiante ou au moins à la température à laquelle l'oxygène ou le gaz contenant de l'oxygène est introduit dans le mélange réactionnel.

4. Un procédé selon la revendication 1, caractérisé en ce que la quantité d'ammoniac est comprise entre 1 et 300, en particulier entre 2 et 80 ppm en poids, par rapport au poids du mélange réactionnel présent dans le réacteur.

5. Un procédé selon l'une des revendications 1—4, caractérisé en ce que dans le cas de l'élévation incontrôlée de température, des systèmes de refroidissement présents dans le réacteur sont aussi mis en marche et/ou on réduit ou on interrompt l'alimentation en oxygène ou en gaz contenant de l'oxygène.

**Patentansprüche**

1. Eine Methode zur Verhütung von Störungen und/oder der Auswirkungen von Störungen bei der Herstellung von Kohlenwasserstoffhydroperoxiden durch Oxidation von Kohlenwasserstoffen mit molekularem Sauerstoff oder einem molekularen Sauerstoff enthaltenden Gas bei erhöhter Temperatur, dadurch gekennzeichnet, daß, wenn ein unkontrollierter Temperaturanstieg bei der Durchführung der Oxidation erfolgt, $NH_3$ in die Reaktionsmischung eingespeist wird.

2. Eine Methode wie in Anspruch 1 beansprucht, dadurch gekennzeichnet, daß das $NH_3$ dem Sauerstoff oder dem Sauerstoff enthaltenden Gas zugesetzt wird.

3. Eine Methode wie in Anspruch 2 beansprucht, dadurch gekennzeichnet, daß das $NH_3$ bei Raumtemperatur oder mindestens bei der Temperatur, bei welcher der Sauerstoff oder das Sauerstoff enthaltende Gas in die Reaktionsmischung eingespeist wird, dampfförmig ist.

4. Eine Methode wie in Anspruch 1 beansprucht, dadurch gekennzeichnet, daß die Menge an $NH_3$ zwischen 1 und 300 und insbesondere zwischen 2 und 80 Gewichtsteile pro Million beträgt, bezogen auf das Gewicht der im Reaktor vorhandenen Reaktionsmischung.

5. Eine Methode wie in Anspruch 1 bis 4 beansprucht, dadurch gekennzeichnet, daß im Fall eines unkontrollierten Temperaturanstieges im Reaktor vorhandene Kühlsysteme gleichfalls in Betrieb gesetzt werden und/oder die Zufuhr von Sauerstoff oder den Sauerstoff enthaltendem Gas verringert oder ganz gestoppt wird.